Europäisches Patentamt

(19) European Patent Office

Office européen des brevets

(11) Publication number: **0 133 200 B1**

# (12) EUROPEAN PATENT SPECIFICATION

(45) Date of publication of patent specification: **25.11.87**

(51) Int. Cl.⁴: **G 01 N 33/531, C 12 Q 1/70, C 12 N 7/02, A 61 K 39/245**

(21) Application number: **84105690.6**

(22) Date of filing: **18.05.84**

(54) Diagnostic antigen for swine pseudorabies disease virus carriers.

(30) Priority: **31.05.83 US 499204**

(43) Date of publication of application:
**20.02.85 Bulletin 85/08**

(45) Publication of the grant of the patent:
**25.11.87 Bulletin 87/48**

(84) Designated Contracting States:
**AT CH DE FR GB LI NL SE**

(56) References cited:
**EP-A-0 069 407**
**EP-A-0 115 442**

**BIOLOGICAL ABSTRACTS, vol.61, no.1, January 1976. J.B.MC FERRAN et al.: "Studies on immunisation of pigs with Barth strain of Aujeszky's disease virus", abstract-no.2512, page 260, Philadelphia (US)**

(73) Proprietor: **Iowa State University Research Foundation, Inc.**
**315 Beardshear**
**Ames, Iowa 50011 (US)**

(72) Inventor: **Platt, Kenneth B.**
**307 North Franklin Street**
**Ames Iowa 50010 (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath**
**Maximilianstrasse 58**
**D-8000 München 22 (DE)**

(56) References cited:
**VETERINARY MICROBIOLOGY, vol.1982, 515-534, Amsterdam; K.B.PLATT "The porcine humoral response to detergent extracted Aujeszky's disease (pseudorabies) virus antigens, pages 515-534**

## Description

The field of this invention relates to diagnostic antigen preparations derived from pseudorabies (PR) virus which are useful in testing the serum of swine to determine whether the swine are carriers of PR virus.

Heretofore a diagnostic preparation for determining swine infection by PR virus has been prepared by solubilizing PR virus-infected cells with an aqueous solution of a nonionic detergent. This preparation contains all of the antigens of the virus. When used in a standard test procedure for the presence of serum antibodies to PR virus, such as the enzyme-linked immunosorbant assay (ELISA), swine found positive are assumed to be actual or potential carriers of the virus. However, positive reaction may be due to natural infection and recovery, or to vaccine immunization with whole killed virus, and the swine may not be carriers of the virus. A negative result, however, does permit the determination that the swine are non-carriers.

Sub-unit vaccines for pseudorabies have been developed and shown to be effective. These vaccines are prepared to contain less than the full complement of antigens from the virus, and, specifically, only the glycoprotein antigens of the virus. One method for preparing such a sub-unit vaccine is described in co-pending application No. WO 84/01290, filed October 5, 1982, and entitled "Lectin-Containing Anti-Viral Vaccines for Domestic Animals and Method of Preparation". Example I of the cited application relates specifically to a vaccine of this kind for pseudorabies. A sub-unit vaccine for pseudorabies containing similar glycoprotein antigens can also be prepared by the method described in co-pending application, No. WO 84/01289, filed October 5, 1982, and entitled "Purified and Antigenically Selective Vaccines for Domestic Animals". Particular reference may be had to Examples I and II of the last-cited application. But it should be understood that the disclosures of these applications are cited only by way of background, and that the details of preparing such sub-unit lectin-binding glycoprotein vaccines for pseudorabies are not an essential part of the disclosure of the present invention. However, it is one of the important advantages of the diagnostic antigenic preparation of this invention that it can be used as a complementary antigenic preparation with respect to the sub-unit vaccines of the above-cited applications, or of other sub-unit vaccines which are based on lectin-binding glycoproteins of PR virus.

In a prior publication, are disclosed studies of the pseudorabies virus antigens. Platt, K. B., *Vet. Micro.*, 7 (1982) 515—534. Pseudorabies virus, also known as Aujeszky's disease virus, was solubilized with an aqueous solution of a non-ionic detergent. The full complement of antigens was studied and partially characterized, the antigens being divided into Groups I, II, and III (see page 521). In separate experiments of the same study, the antigens which were adsorbed on a lectin-agarose gel column (the lectin retentate antigens) were studied. It was found that these were antigens of Group III (see page 524). Although the cited publication does not discuss diagnostic antigenic preparations for pseudorabies, it would be apparent to those skilled in the viral vaccine art that a diagnostic preparation containing only Group I antigens, for example, might be useful in conjunction with a sub-unit vaccine containing only Group III antigens. However, prior to the present invention no procedure was known for obtaining such a diagnostic antigenic preparation without the expensive and cumbersome procedure of solubilizing all of the AD virus antigens and separating the antigens by a protein fractionation procedure such as a chromatographic column separation.

Also, the European patent application 0 115 442 does not describe the recovery and separation of the antigens other than as known in the prior art.

This invention is based on the discovery of a simple and convenient procedure for producing a diagnostic antigen for use as a complementary antigen with sub-unit vaccines for pseudorabies which contain lectin-binding glycoprotein antigens. Diagnostic antigens of the class desired have been found to be elaborated into the extracellular medium in which PR virus-infected cells are cultured for the propagation of the virus, such as in the production of vaccines. This discovery therefore makes it possible to obtain a complementary diagnostic antigen preparation as a by-product of the manufacture of sub-unit vaccines of the kind described above.

The medium-elaborated antigenic factor (MEA) of this invention has not been fully characterized. However, the antigenic factors of this preparation have been shown to be like the Class I antigen group, as described in prior publication (*Vet. Micro.*, 7 (1982), 515—534). The Rf value of MEA as determined against human albumin in the absence of urea is about 1.0. Further, the antigenic factors of MEA are clearly different from the antigens of Groups II and III. The MEA may contain more than one protein, or may be a monomer or polymer of a single protein. It is believed that MEA contains precursor proteins for the formation of the Group I proteins of the AD virus, although this has not been definitely established. Remarkably, however, MEA is the only antigenic factor related to the PR virus found in the medium external to the cells, if the medium is harvested from PR virus, that is, there are no detectable antigens of Groups II and III present, providing that the medium is harvested at an intermediate time during the viral replication cycle. The thus-harvested medium is therefore an ideal source for a complementary diagnostic antigen. Further, it can readily be removed and replaced during the culturing of the cells to produce virus.

The method of this invention can be carried out by using known culturing and viral replication procedures for PR virus. Such procedures have heretofore been described in the literature. See Platt, et al, *Archives Virolog.*, 60 (1979), 13—23).

For example, a standard roller bottle apparatus may be used. A cell-line is selected, such as kidney cells adapted for in vitro propagation, the cells being ones in which PR virus replicate freely. The cells are introduced into the roller bottles together with a suitable aqueous medium, such as Eagle's minimum essential medium. During the initial growth of the cells it is preferred, as in prior practice to incorporate fetal calf serum in the medium, such as at a level of 10% by volume. After the cells have been grown to form a monolayer in the bottles, the medium is removed, and the cells are infected with an inoculant solution containing live PR virus. The inoculant is left in contact with the cells for a sufficient time to obtain infection, such as about 90 minutes, and is then removed. The infected cells in the monolayer are then washed, with Earle's medium without FCS. After removal of the wash solution, additional medium free of FCS, such as Earle's medium, is added. The infected cells are incubated in contact with the medium according to the usual practice until the viral replication has proceeded to an intermediate time in the viral replication cycle where the desired diagnostic antigenic factor (MEA) has been released from the cells into the medium. At that time, the incubation is interrupted, and the medium is removed to obtain the aqueous solution of the MEA for use as a diagnostic antigen. If desired, as will usually be the case, additional medium is then added, and the incubation is continued to the completion of the viral replication cycle. The virus can then be harvested in the usual way and employed to prepare vaccine preparations, such as particularly sub-unit vaccines containing the lectin-binding glycoprotein antigens, as described above.

Cell lines of adapted swine or bovine kidney cells have been found particularly desirable for replication of PR virus. One suitable cell line is the PK-15 cell line obtainable from the National Veterinary Services Laboratory in Ames, Iowa. Another suitable cell line is the MDBK cell line which is obtainable from the American type Culture Collection at Rockville, Maryland, under Accession No. CCL-22. However, any cell line can be employed in which the PR virus replicate.

The optimum time, for harvesting MEA, can easily be determined in relation to an established culturing procedure. For example, with an infection multiple of 5, the medium can be advantageously collected around 6 hours after initiation of the infection. Where a multiple of 10 infectious virus particles per cell is employed, it appears that the optimum time for collecting the medium will be around 4 hours. In general, the cells may be infected with multiples of from 3 to 12 virus per cell, and the medium containing the antigen may be harvested within the time period from 2 to 8 hours after the start of the replication. It appears that the optimum time for antigen harvest is usually 4 to 6 hours after infection of the cells, but the exact time of maximum yield will be determined in part by the multiplicity of virus infection used. Further, if too high a multiplicity is used it is difficult to obtain the MEA in relatively pure form, other antigens of Group II and III being present in admixture with MEA. Therefore, it is preferred to use a low multiplicity such as a multiplicity of 5 or less and to collect the MEA at about 6 hours post cell infection. It should be understood that while these infection levels and collection times are set out as preferred examples, the method of this invention is not limited thereto.

Incubation conditions both for the growing of the cells and for the replication of the virus may be those which are usually employed, for example, an incubation temperature of around 37°C.

After separation of the MEA-containing aqueous medium from the cells, some further processing steps are desirable, although not essential. For example, the medium may be centrifuged or filtered to remove cell debris. If desired, the centrifugation may be under very high gravity, effective for pelleting virus. Residual virus in the medium can thereby also be removed. Further, to be sure that no live virus carry through in the diagnostic antigen, it can be heated at a suitable temperature and time for killing the virus, such as at a temperature of 56°C for one hour. Such heating inactivates the PR virus but does not significantly denature the MEA protein.

Following the solids removal and heat treatment as described, the solution of the MEA may be concentrated, if desired, such as by ultrafiltration. Concentration is not required, however, since the medium from a single roller bottle will contain sufficient MEA for several hundred assays.

The MEA preparation described above can be used in standard immunoassay procedures, such as particularly the ELISA procedure. Such assay procedures, however, are not part of the present invention, and will be carried out in accordance with known practice.

The method of this invention and the MEA antigenic preparation obtained thereby is further illustrated by the following experimental examples.

Example I

The diagnostic antigen of this invention (MEA) is prepared as follows:

1. Confluent cell monolayers are prepared in plastic roller bottles. The cell type used is PK-15 or the MDBK cell lines. Other cell types that support the replication of pseudorabies virus may also be used.

2. The cell monolayers are inoculated with pseudorabies virus at a multiplicity of infection of 1 to 5 and incubated at 37°C for 90 minutes.

3. The virus inoculum is then removed from the cell monolayers.

4. The cell monolayers are washed three times with minimum essential medium containing antibiotics and free of serum supplement.

5. The washed monolayers are then supplied with serum-free minimum essential medium containing antibiotic. The medium is supplied at a rate of 40 ml per 850 cm$^2$ roller bottle.

6. The infected cell monolayers are then reincubated at 37°C.

7. The serum-free medium containing the MEA is removed from the cell containers between 4 and 6 hours following virus inoculation. The optimum time for harvesting must be determined by trial and error.

8. New serum-free medium is added to the cell monolayers which are ultimately used as a source of antigen for the subunit vaccines described earlier.

9. The harvested MEA is clarified by low speed centrifugation then heated to 56°C and maintained at this temperature for 30 to 60 minutes for the purpose of destroying potential virus activity.

10. The MEA is then filtered through a 220 nm pore size Milipore filter.

11. The filtrate is then concentrated by ultrafiltration for ease in storage or used directly in the undiluted state as diagnostic antigen.

Example II

MEA antigen prepared as described in Example I can be employed in enzyme-linked immunosorbent assays (ELISA) as follows:

1. One hundred µl of optimally diluted MEA in 0.02M carbonate/bicarbonate buffer pH 9.6 is added to alternate rows of individual wells of polyvinyl chloride (PVC) Microtiter plates (Dynatech Laboratories, Alexandria, VA).

The same amount of sham antigen consisting of maintenance medium of non-virus infected cells is then added to the remaining rows of empty wells.

The correct dilution of sham antigen to be used is that dilution which when added to wells and ultimately reacted with known negative serum gives the same absorbance reading as that which occurs when the same negative serum is reacted with MEA.

2. The PVC plates are then incubated 3 hours at 37°C for at least 18 h at 4°C to permit the antigen to adsorb to the plate.

3. Immediately prior to use the antigen coated plates are washed 3 times with phosphate buffered saline 0.01M, pH 7.2 containing 0.05% Tween 20.

4. Test and appropriate control serums are diluted 1:20 in 0.05M Tris buffer pH 7.4 containing 150 mM NaCl, 0.01 mM EDTA, 0.05% Tween 20 and 1.0% bovine serum albumin.

5. One hundred µl of individually diluted serums are added to MEA-coated and sham antigen-coated wells in duplicate.

6. Plates are then incubated at 37°C for 30 min and subsequently washed with PBS.

7. Fifty µl of goat anti-porcine IgG conjugated to horseradish peroxidase is added to individual wells and the plates are reincubated at 25°C for 30 minutes.

8. The plates are then washed with PBS and 100 µl of enzyme substrate is added to individual wells. The enzyme substrate is prepared by adding 400 µl of 1% hydrogen peroxide and 100 µl of a 40 mM solution of 2-2'azino-di-(3-ethylbenzthiazoline-6-sulfonate) to 10 ml of 0.05M citric acid pH 4.0.

9. The color reaction following the addition of enzyme substrate is allowed to develop at 25°C for 20 minutes and then stabilized by adding 50 µl of a 1:400 dilution of 48% concentrated reagent grade hydrofluoric acid.

10. The mean absorbance value for the duplicate wells containing MEA and the sham antigen is determined with the Dynatech MR 580 ELISA microplate reader using a test filter wavelength of 405 nm and a reference filter wavelength of 405 nm.

11. The ratio of the mean absorbance values of an individual serum reacting with MEA to the mean absorbance value obtained from reacting the same serum with sham antigen was determined. Ratios of 1.75 or greater are considered to indicate the presence of antibody to MEA.

Example III

This experiment demonstrates the uniqueness of MEA as an antigen to be used in the ELISA for the differentiation of vaccinated pigs that have been infected with pseudorabies virus from vaccinated pigs that have not been infected with the virus. The MEA was prepared as described in Example I and the ELISA tests were carried out as described in Example II.

Twelve pigs were vaccinated with 2 doses of a subunit vaccine consisting of lectin-bound glycoprotein antigens. Ten pigs from the same litters were maintained as uninoculated controls. Three weeks later the second vaccine dose was given and sera were collected from all pigs. Each pig was then challenged intranasally with $10^{8.5}$ plaque forming units of virulent PR virus. Eleven of the 12 vaccinated pigs survived the challenge. In contrast all 10 control pigs died within eight days of challenge.

Serums were collected from all survivors on the day the first vaccine dose was given, on the day of virus challenge, and at 10, 21 and 62 days post challenge. Each serum sample was then assayed for the presence of PR virus specific antibody by the ELISA test utilizing (a) the MEA and (b) conventional antigen prepared by solubilizing whole virus-infected cells. The principle difference between these antigens is that the MEA does not contain antigens that are present in the subunit vaccine whereas the conventional antigen does contain the antigens found in the vaccine.

The results of the ELISA tests utilizing each antigen is summarized in Table A. Test values (i.e., absorbance ratios) ranging up to 1.75 are considered negative. Values that exceed 1.75 indicate the presence of antibody specific for PR virus antigens. These data indicate that vaccinates were free of antibody on the day [-42] that they received the first vaccine dose. The data also shows that vaccinates were still negative for PR virus antibody on the day of virus challenge (day 0) when the ELISA test was run with MEA. However, when the ELISA test was run with conventional antigen PR virus specific antibody was detected in all vaccinates, i.e., absorbance ratios exceeded 1.75. Following challenge, antibody to PR virus was detected in the vaccinates by the ELISA test when either MEA or conventional antigen was used.

These results clearly demonstrate that MEA can be used to detect PR virus infections in the pig. Furthermore, because MEA does not contain vaccine antigen it can be used to differentiate virus-infected subunit-vaccinated pigs from subunit-vaccinated pigs that have not been infected with the virus.

TABLE A

Antibody response of vaccinated and control pigs before and after pseudorabies virus challenge as determined by the ELISA with MEA and conventional diagnostic antigen

| Pigs | Antigen | Days[a] pre and post challenge | | | | |
|---|---|---|---|---|---|---|
| | | −42 | 0 | 10 | 21 | 62 |
| Vaccinates[b] | MEA | 1.1 (±.2)[c] | 1.1 (±.1) | 5.1 (±.7) | 5.4 (±.3) | 3.8 (±.2) |
| | Conventional | 1.1 (±.1) | 3.9 (±.3) | 6.4 (±.9) | 6.5 (±.2) | 6.6 (±.6) |
| Controls | MEA | — | 1.1 (±.1) | — | — | — |
| | Conventional | — | 1.0 (±.1) | — | — | — |

[a] Day −42 is the day that the vaccinates received the first vaccine dose. Day 0 is the day that all pigs were nasally challenged with pseudorabies virus.
[b] N=12 for vaccinates through day 0, then N=11. N=10 for controls.
[c] Values=the ratio of the absorbance obtained with MEA or conventional antigen to the absorbance obtained with sham antigen. Ratios >1.75 indicate the presence of pseudorabies-specific antibody.

## Claims

1. A method of preparing a diagnostic medium-elaborated antigenic protein (MEA) for use in determining swine carriers of pseudorabies disease virus (PR) virus, wherein cells are cultured which are adapted to *in vitro* propagation and in which PR virus will replicate, the cultured cells are infected with live PR virus, and the virus are replicated in the infected cells in contact with a detergent-free aqueous culture medium, characterized by the steps of interrupting the PR virus replication at an intermediate time in the replication cycle following the cell infection when said medium contains said MEA in essentially antigenically pure form with reference to other antigenic proteins of the PR virus, and separating the culture medium from the intact cells to obtain an aqueous solution of MEA.

2. The method of claim 1 in which the cell culture is infected with from 3 to 12 live PR virus per cell.

3. The method of claim 1 or claim 2 in which said culture is continued for at least two hours but not over 8 hours before the culture medium is separated to obtain the MEA.

4. The method for testing swine serum to determine the presence of antibodies to pseudorabies (PR) virus as distinguished from antibodies to lectin-binding PR glycoproteins wherein an immunoassay is performed on the swine serum using a PR virus antigen preparation, characterized by employing as said antigen preparation the MEA product produced according to the method of claims 1, 2 or 3.

5. The testing method of claim 4 in which said immunoassay is an enzyme-linked immunosorbent assay (ELISA).

## Patentansprüche

1. Verfahren zum Herstellen eines diagnostischen, in das Medium ausgeschiedenen antigenen Proteins (MEA) für die Verwendung zum Bestimmen von porkinen Trägern des Pseudorabiesvirus (PR-Virus), wobei Zellen kultiviert werden, die an *in vitro* Propagation adaptiert sind und in denen das PR-Virus replizieren kann, wobei die kultivierten Zellen mit lebendem PR-Virus infiziert werden und die Viren in den infizierten Zellen, die in Kontakt mit einem Detergenz-freien wässrigen Kulturmedium stehen, repliziert werden, gekennzeichnet durch den Schritt des Unterbrechens der PR-Virus-Replikation zu einer in dem Replikationszyklus liegenden Zwischenzeit nach der Zellinfektion, wenn das besagte Medium das besagte MEA in im wesentlichen reiner antigener Form enthält im Hinblick auf andere antigene Proteine des PR-Virus und durch den Schritt des Trennens des Kulturmediums von intakten Zellen, um eine wässrige Lösung von MEA zu erhalten.

2. Verfahren nach Anspruch 1, in dem die Zellkultur mit drei bis zwölf lebenden PR-Viren pro Zelle infiziert wird.

3. Verfahren nach Anspruch 1 oder 2, in dem die besagte Kultur mindestens zwei Stunden, aber nicht mehr als acht Stunden fortgeführt wird, bevor das Kulturmedium abgetrennt wird, um das MEA zu erhalten.

4. Verfahren zum Untersuchen von Schweineserum, um die Anwesenheit von Antikörpern gegen Pseudorabies (PR)-Virus im Unterschied zu Antikörpern gegen Lektin-bindende PR-Glycoprotein zu bestimmen, in dem ein Immunoassay mit dem Schweineserum unter Verwendung einer PR-Virus-Antigen-Präparation durchgeführt wird, gekennzeichnet durch die Anwendung des gemäß dem Verfahren der Ansprüche 1, 2 oder 3 hergestellten MEA-Produktes als besagte Antigenpräparation.

5. Verfahren nach Anspruch 4, in dem der genannten Immunoassay ein Enzym-gebundener Immunadsorptionstest (ELISA) ist.

**Revendications**

1. Procédé pour préparer une protéine antigène élaborée en milieu (MEA) diagnostique servant à déterminer chez les porcs ceux qui sont porteurs du virus responsable de la pseudorabies (PR), dans lequel on cultive des cellules qui sont adaptées à la propagation *in vitro* et dans lesquelles le virus PR se réplique, on infecte les cellules de culture avec le virus PR vivant, et les virus se répliquent dans les cellules infectées en contact avec un milieu de culture aqueux dépourvu de détergent, caractérisé en ce qu'on interrompt la réplication des virus PR à un instant intermédiaire dans le cycle de réplication après l'infection des cellules lorsque ledit milieu contient la MEA sous forme pratiquement antigéniquement pure par référence à d'autres protéines du virus PR, et ce qu'on sépare le milieu de culture des cellules intactes pour obtenir une solution aqueuse de MEA.

2. Procédé selon la revendication 1, dans lequel la culture de cellules est infectée sur la base de 3 à 12 virus PR vivants par cellule.

3. Procédé selon la revendication 1 ou la revendication 2, dans lequel la culture se prolonge pendant au moins deux heures, mais pas plus de huit heures, avant la séparation du milieu de culture pour obtenir la MEA.

4. Procédé de contrôle du sérum de porcs pour déterminer la présence d'anticorps du virus de la pseudorabies (virus PR), en les distinguant d'anticorps des glyco-protéines de la PR liant la lectine, dans lequel on effectue un test immunologique sur le sérum de porc en utilisant une préparation d'antigènes du virus de la PR, caractérisé en ce qu'on emploie comme préparation d'antigènes la préparation de MEA préparée selon le procédé des revendications 1 à 3.

5. Procédé de contrôle selon la revendication 4, dans lequel ce test immunologique est un test immunoenzymatique par sorption (ELISA).

6